# EUROPEAN PATENT SPECIFICATION

(11) **EP 2 056 874 B1**
(45) Date of publication and mention of the grant of the patent: **19.09.2012**
(21) Application number: 07786693.7
(22) Date of filing: 17.08.2007
(51) Int. Cl.: A61K 39/395, C07K 16/00

(54) **TUMOR THERAPY WITH AN ANTI-VEGF ANTIBODY**
TUMORTHERAPIE MIT EINEM ANTI-VEGF-ANTIKÖRPER
THÉRAPIE TUMORALE AVEC ANTICORPS ANTI-VEGF

(30) Priority: 21.08.2006 EP 06017330
(43) Date of publication of application: 13.05.2009
(60) Divisional application: 11193793.4
(73) Proprietor: F. Hoffmann-La Roche AG, 4070 Basel (CH)
(72) Inventor: FRIESS, Thomas, 86911 Diessen-Dettenhofen (DE); HASMANN, Max, 81377 Muenchen (DE); SCHEUER, Werner, 82377 Penzberg (DE)
(74) Representative: Burger, Alexander
(86) International application number: PCT/EP2007/007276
(87) International publication number: WO 2008/022746

(56) References cited:
- WO-A-2007/107329
- PEGRAM M D ET AL: "Phase I combined biological therapy of breast cancer using two humanized monoclonal antibodies directed against HER2 proto-oncogene and vascular endothelial growth factor (VEGF)" BREAST CANCER RESEARCH AND TREATMENT, NIJHOFF, BOSTON, US, vol. 88, no. Suppl 1, 2004, pages S124-S125, XP002384467 ISSN: 0167-6806
- PEGRAM M K D ET AL: "Combined biological therapy of breast cancer using monoclonal antibodies directed against HER2/neu protein and vascular endothelial growth factor" SEMINARS IN ONCOLOGY, W.B. SAUNDERS,, US, vol. 29, no. 3, SUPPL 11, June 2002 (2002-06), pages 29-37, XP009067550 ISSN: 0093-7754
- MANOIR DU J M ET AL: "Strategies for delaying or treating in vivo acquired resistance to trastuzumab in human breast cancer xenografts" CLINICAL CANCER RESEARCH, THE AMERICAN ASSOCIATION FOR CANCER RESEARCH, US, vol. 12, no. 3 Pt 1, 1 February 2006 (2006-02-01), pages 904-916, XP002384468 ISSN: 1078-0432
- EPSTEIN M ET AL: "HER2-overexpressing human breast cancer xenografts exhibit increased angiogenic potential mediated by vascular endothelial growth factor (VEGF)." BREAST CANCER RESEARCH AND TREATMENT, vol. 76, no. Supplement 1, December 2002 (2002-12), page S143, XP002416579 & 25TH ANNUAL SAN ANTONIO BREAST CANCER SYMPOSIUM; SAN ANTONIO, TX, USA; DECEMBER 11-14, 2002 ISSN: 0167-6806
- PEGRAM M ET AL: "Phase II combined biological therapy targeting the HER2 proto-oncogene and the vascular endothelial growth factor using trastuzumab (T) and bevacizumab (B) as first line treatment of HER2-amplified breast cancer." BREAST CANCER RESEARCH AND TREATMENT, vol. 100, no. Suppl. 1, 2006, pages S28-S29, XP002455370 & 29TH ANNUAL SAN ANTONIO BREAST CANCER SYMPOSIUM; SAN ANTONIO, TX, USA; DECEMBER 14 -17, 2006 ISSN: 0167-6806
- ANONYMOUS: "Combination of herceptin and avastin shows premise in treatment of aggressive form of breast cancer"[Online] 15 December 2006 (2006-12-15), XP002455371 Retrieved from the Internet: URL:http://www.druginfozone.nhs.uk/documen ts/herceptin%20avastin%20press%20release.d oc?id=574193> [retrieved on 2007-10-17]
- SCHEUER W ET AL: "Enhanced antitumour effect by combination of HER2-targeting antibodies with bevacizumab in a human breast cancer xenograft model" EJC SUPPLEMENTS, vol. 4, no. 12, November 2006 (2006-11), page 66, XP002455372 & 18TH SYMPOSIUM ON MOLECULAR TARGETS AND CANCER THERAPEUTICS; PRAGUE, CZECH REPUBLIC; NOVEMBER 07 -10, 2006 ISSN: 1359-6349

## Description

The present invention is directed to the treatment of a patient suffering from relapsed HER2 positive cancer with an anti-VEGF antibody during or after treatment with an anti-HER2 antibody.

### Background of the Invention

Angiogenesis is implicated in the pathogenesis of a variety of disorders which include solid tumors, intraocular neovascular syndromes such as proliferative retinopathies or age-related macular degeneration (AMD), rheumatoid arthritis, and psoriasis (Folkman et al., J. Biol. Chem. 267 (1992) 10931- 10934; Klagsbrun et al., Annu. Rev. Physiol. 53 (1991) 217-239; and Garner, A., Vascular diseases, In: Pathobiology of ocular disease, A dynamic approach, Garner, A., and Klintworth, G. K. (eds.), 2nd edition, Marcel Dekker, New York (1994), pp 1625-1710). In the case of solid tumors, the neovascularization allows the tumor cells to acquire a growth advantage and proliferative autonomy compared to the normal cells. Accordingly, a correlation has been observed between density of microvessels in tumor sections and patient survival in breast cancer as well as in several other tumors (Weidner et al., N. Engl. J. Med. 324 (1991) 1-8; Horak et al., Lancet 340 (1992) 1120-1124; and Macchiarini et al., Lancet 340 (1992) 145-146).

Vascular endothelial growth factor (VEGF) is involved in the regulation of normal and abnormal angiogenesis and neovascularization associated with tumors and intraocular disorders (Ferrara et al., Endocr. Rev. 18 (1997) 4-25; Berkman et al., J. Clin. Invest. 91 (1993) 153-159; Brown et al., Human Pathol. 26 (1995) 86-91; Brown et al., Cancer Res. 53 (1993) 4727-4735; Mattern et al., Brit. J. Cancer. 73 (1996) 931-934; and Dvorak et al., Am. J. Pathol. 146 (1995) 1029-1039). Anti-VEGF neutralizing antibodies suppress the growth of a variety of human tumor cell lines in mice (Kim et al., Nature 362 (1993) 841-844; Warren et al., J. Clin. Invest. 95 (1995) 1789-1797; Borgstrom et al., Cancer Res. 56 (1996) 4032-4039; and Melnyk et al., Cancer Res. 56 (1996) 921-924). WO 94/10202, WO 98/45332, WO 2005/00900 and WO 00/35956 refer to antibodies against VEGF. Humanized monoclonal antibody bevacizumab (sold under the tradename Avastin^{®}) is an anti-VEGF antibody used in tumor therapy and is the only anti-angiogenic agent approved for treatment of cancer (WO 98/45331).

HER2 is a member of the human epidermal growth factor receptor family and possesses protein kinase activity in its cytoplasmic domain. HER2 is over-expressed in tumor cells and is correlated with poor prognosis and survival. HER2 is therefore a valuable target of breast cancer therapy. Antibodies against HER2 are known from Takai N. et al., Cancer 104 (2005) 2701-2708; Yeon, C. H., et al., Invest New Drugs. 23 (2005) 391-409; Wong, W. M., et al, Cancer Pract. 7 (1999) 48-50; Albanell, J., et al., Drugs Today (Barc). 35 (1999) 931-46.

Trastuzumab (sold under the tradename Herceptin^{®}) is a recombinant humanized anti-HER2 monoclonal antibody used for the treatment of HER2 over-expressed/HER2 gene amplified metastatic breast cancer. Preclinical studies demonstrated that the antibody has anti-tumor activity in vivo and in vitro. Moreover, additive or synergistic enhancement of anti-tumor activity of trastuzumab was observed in combination with various anti-tumor agents in mouse models. In clinical studies, extension of survival was observed in HER2 overexpressing metastatic breast cancer patients.

WO 2005/012531 describes antibodies that may be combined with anti-ErbB antibodies (e.g. Herceptin^{®} also known as trastuzumab) and/or an anti-VEGF antibody (e.g. Avastin^{®} also known as bevacizumab) in the treatment of colorectal cancer, metastatic breast cancer and kidney cancer. According to WO 2005/063816, anti-VEGF antibodies may be combined with anti-ErbB antibodies in a treatment of metastatic breast cancer. According to WO 98/45331, the effectiveness of an anti-VEGF antibody in preventing or treating disease may be improved by administering the antibody serially or in combination with another agent that is effective for those purposes, such as an antibody capable of binding to HER2 receptor. WO 2005/00090 and WO 2003/077841 also disclose the combination of anti-VEGF antibodies with anti-ErbB2 antibodies for tumor therapy. Pegram, M.D., et al, Seminars in Oncology 29 (2002) 29-37, relates to the combination of anti-ErbB2 antibodies with anti-VEGF antibodies in the therapy breast cancer.

Clinical oncologists are in agreement that the failure of cancer treatment is not necessarily caused by the growth of the primary tumor, which is generally dealt with using surgery, but rather by the metastatic spread into different organs. The regression of primary tumors by different cytotoxic drugs is not always indicative for anti-metastatic activity per se. On the contrary, enhanced metastasis has been observed in response to several anti-cancer drugs (Geldof et al., Anticancer Res 8 (1988) 1335-40, Murphy, J., Clin. Oncol. 11 (1993) 199-201, and De Larco et al., Cancer Res. 61 (2001) 2857-61). Clearly there exists a need to develop treatment therapies that target not only the primary tumor, but also suppress metastasis.

These anti-metastatic activities can e.g. be evaluated by the method according to Schneider, T., et al, Clin. Exp. Metas. 19 (2002) 571-582.

### Summary of the Invention

The invention comprises the claim 1.

A further embodiment is claim 8.

Preferably said anti-VEGF antibody binds to the same epitope as bevacizumab.

Preferably said anti-VEGF antibody is bevacizumab.

Preferably said anti-HER2 antibody is trastuzumab.

In a preferred embodiment, the invention comprises co-administering said anti-VEGF antibody and said anti-HER2 antibody to the patient.

### Detailed Description of the Invention

The term "VEGF" according to the invention refers to the vascular endothelial cell growth factor (Swiss-Prot No. P 15692), alternative splicing forms (see e.g. Leung et al., Science, 246 (1989) 1306-1309, and Houck et al., Mol. Endocrin., 5 (1991) 1806-14 and active fragments, preferably N-terminal fragments thereof.

The term "anti-VEGF antibody" according to the invention is an antibody that binds specifically to VEGF and exhibit an antiangiogenetic activity. The preferred humanized anti-VEGF antibody or variant anti-VEGF antibody herein binds human VEGF with a Kd value of no more than about 1 × 10⁻⁸M and preferably no more than about 5×10⁻⁹M. Preferably the anti-VEGF antibody is a monoclonal antibody that binds to the same epitope as recombinant humanized anti-VEGF monoclonal antibody (bevacizumab) generated according to Presta et al., Cancer Res. 57 (1997) 4593-4599. A preferred antibody is bevacizumab. Anti-VEGF antibodies and methods for their manufacture are e.g. described in US 6,054,297, US2003/0190317, US 6,632,926, US 6,884,879, and US 2005/0112126.

Bevacizumab comprises mutated human IgG1 framework regions and antigen-binding complementarity-determining regions from a murine anti-hVEGF monoclonal antibody that blocks binding of human VEGF to its receptors. Approximately 93% of the amino acid sequence of bevacizumab, including most of the framework regions, is derived from human IgG1, and about 7% of the sequence is derived from the murine antibody A4.6.1. Bevacizumab has a molecular mass of about 149,000 daltons and is glycosylated. Bevacizumab and its method of preparation are described in EP 1325932.

The term "HER2" according to the invention refers to 185-kDa growth factor receptor also referred to as neu and c-erbB-2 (Slamon et al., Science 235 (1987) 177-182; Swiss-Prot P04626) whose function is related to neoplastic transformation in human breast cancer cells. Overexpression of this protein has been identified in 20-30% of breast cancer patients where it correlates with regionally advanced disease, increased probability of tumor recurrence, and reduced patient survival. As many as 30-40% of patients having gastric, endometrial, salivary gland, non-small cell lung, pancreatic, ovarian, peritoneal, prostate, or colorectal cancers may also exhibit overexpression of this protein.

The term "anti-HER2 antibody" according to the invention is an antibody that binds specifically to the same epitope of HER2 as the murine anti-HER2 antibody 4D5 described in Hudziak et al., Mol. Cell. BioL 9 (1989) 1165-1172. Anti-HER2 antibodies which bind to the "4D5 epitope of HER2", including anti-HER2 antibody 4D5 itself, and methods for their manufacture are e.g. described in US 6,054,297, WO 89/06692, US 6,399,063, US 6,165,464, US 6,054,297, US 5,772,997, WO 2003/087131, WO 01/00245, WO 01/00238, WO 00/69460, WO 99/31140 and WO 98/17797. In a preferred embodiment of the invention, the anti-HER2 antibody is trastuzumab, a recombinant humanized anti-HER2 monoclonal antibody (a humanized version of the murine anti-HER2 antibody 4D5, referred to as rhuMAb HER2 or trastuzumab) which has been clinically active in patients with HER2-overexpressing metastatic breast cancers that had received extensive prior anticancer therapy. (Baselga et al, J Clin. Oncol. 14 (1996) 737-744). Trastuzumab and its method of preparation are described in EP 590058.

The "epitope 4D5" is the region in the extracellular domain of ErbB2 to which the antibody 4D5 (ATCC CRL 10463) binds. This epitope is close to the transmembrane region of ErbB2. To screen for antibodies which bind to the 4D5 epitope, a routine cross-blocking assay such as that described in Antibodies, A Laboratory Manual, Cold Spring Harbor Laboratory, Ed. Harlow and David Lane (1988), can be performed. Alternatively, epitope mapping can be performed to assess whether the antibody binds to the 4D5 epitope of ErbB2.

The term "epitope" as used within this application denotes a protein determinant capable of specific binding to an antibody. Epitopes usually consist of chemically active surface groupings of molecules such as amino acids or sugar side chains and usually have specific three dimensional structural characteristics, as well as specific charge characteristics. Conformational and non-conformational epitopes are distinguished in that the binding to the former but not the latter is lost in the presence of denaturing solvents. Depending on the size of the antigen to which the epitope belongs, more than one epitope per antigen may be available resulting likewise in the possibility of more than one antibody binding site (=epitope) per antigen.

Antibodies can be generated against, e.g., human, mouse, or rat polypeptides. Antibodies, either polyclonal or monoclonal, specifically recognizing the target antigen are encompassed by the invention. Such antibodies are raised using standard immunological techniques known to a person skilled in the art. Antibodies may be polyclonal or monoclonal or may be produced recombinantly such as for a humanized antibody. The determination if an antibody is not binding to the same epitope as a known therapeutic antibody can easily be determined in a competitive test system.

Possible epitope overlapping of two antibodies binding to the same target antigen can be detected with the help of a competitive test system. For this purpose, for example with the help of an enzyme immunoassay, there is tested the extent to which the new antibody competes with the known antibody for the binding to an immobilized target antigen. For this purpose, an appropriately immobilized target antigen is incubated with the known antibody in labeled form and an excess of the antibody in question. By detection of the bound labeling there can easily be ascertained the extent to which the antibody in question can displace the known antibody from the binding site (= epitope). If there is a displacement of more than 10%, preferably of more than 20%, at the same concentration or at higher concentrations, preferably in the case of 10⁵-fold excess of the antibody in question, referred to the known antibody, then an epitope overlapping is present. That means that the antibody in question binds to the same epitope as the known antibody.

The term "target antigen" relates to a biomolecule which is bound by its corresponding therapeutic antibody. By way of example, the target antigen of a therapeutic antibody to HER2 (= ErbB2 or p 185 *^{neu}),* like Herceptin^{®} or Omnitarg^{®}, is HER2, of a therapeutic antibody to EGFr, like Erbitux^{®}, is EGFr, of a therapeutic antibody to VEGF, like Avastin^{®}, is VEGF. The target antigen may either be a soluble, i.e. secreted or shed, target antigen or a (cell-)membrane bound target antigen.

Immunoassays are well known to the skilled artisan. Methods for carrying out such assays as well as practical applications and procedures are summarized in related textbooks. Examples of related textbooks are Tijssen, P., Preparation of enzyme-antibody or other enzyme-macromolecule conjugates, In: Practice and theory of enzyme immunoassays, Burdon, R.H. and v. Knippenberg, P.H. (eds.), Elsevier, Amsterdam (1990), pp. 221-278; and various volumes of Colowick, S.P. and Caplan, N.O. (eds.), Methods in Enzymology, Academic Press, dealing with immunological detection methods, especially volumes 70, 73, 74, 84, 92 and 121.

The term "overexpression" of the HER2 receptor protein is intended to indicate an abnormal level of expression of the HER2 receptor protein in a cell from a tumor within a specific tissue or organ of the patient relative to the level of expression in a normal cell from that tissue or organ. Patients having a cancer characterized by overexpression of the HER2 receptor can be determined by standard assays known in the art. Preferably overexpression is measured in fixed cells of frozen or paraffin-embedded tissue sections using immunohistochemical (IHC) detection. When coupled with histological staining, localization of the targeted protein can be determined and extent of its expression within a tumor can be measured both qualitatively and semi-quantitatively. Such IHC detection assays are known in the art and include the Clinical Trial Assay (CTA), the commercially available LabCorp 4D5 test, and the commercially available DAKO HercepTest^{®}(DAKO, Carpinteria, Calif.). The latter assay uses a specific range of 0 to 3+ cell staining (0 being normal expression, 3+ indicating the strongest positive expression) to identify cancers having overexpression of the HER2 protein (see the Herceptin^{®}(trastuzumab) full prescribing information; September 1998; Genentech, Inc., San Francisco, Calif.). Thus, patients having a cancer characterized by overexpression of the HER2 protein in the range of 1+, 2+, or 3+, preferably 2+ or 3+, more preferably 3+ would benefit from the methods of therapy of the present invention.

The term "HER2 positive cancer" refers to a cancer disease such as breast cancer, gastric cancer, endometrial cancer, salivary gland cancer, non-small cell lung cancer, pancreatic cancer, ovarian cancer, peritoneal cancer, prostate cancer, or colorectal cancer, which is characterized by an overexpression of HER2 protein. In accordance with the invention, the cancer is a relapsed HER2 positive breast cancer, which is treated preferably following or after a first-line monotherapy with an anti-HER2 antibody, wherein said anti-HER2 antibody is preferably trastuzumab.

The term "breast cancer" refers to the uncontrolled growth of abnormal breast cells. It includes ductal carcinoma in situ, invasive ductal carcinoma, lobular carcinoma in situ, invasive lobular carcinoma, medullary carcinoma, Paget's disease of the nipple and metastatic breast cancer.

The term "relapsed cancer" refers to the uncontrolled growth of abnormal cells in tumor patients who initially responded to previous therapy, but in whom the therapeutic response was not maintained. The term "relapsed HER2 positive cancer" refers to the uncontrolled growth of abnormal cells characterized by HER2 protein overexpression in tumor patients who initially responded to previous therapy with an anti-HER2 antibody, preferably trastuzumab, but in whom the therapeutic response was not maintained during treatment with said anti-HER2 antibody. Tumor patients who initially responded to previous therapy with an anti-HER2 antibody, preferably trastuzumab, but in whom the therapeutic response was not maintained are referred to as "relapsers".

Therapeutic response (RE) is established based on the medical judgment of a practitioner ascertained by the results from clinical and laboratory data that are generally known in the art to assess patient treatment. Such data may be obtained, by way of example, from clinical examination, cytological and histological techniques, endoscopy and laparoscopy, ultrasound, CT and MRI scans, chest X-ray and mammography, and measuring the concentration of tumor markers, such as CEA, Cyfra, CA15-3, interleukin 8 and soluble HER2. Preferably RECIST criteria may be used to determine tumor response (RE). (Therasse et al., J. Nat. Cancer Institute. 92 (2000) 205-216).

According to these RECIST criteria tumor response for solid tumors (Therasse, et al. J. Nat. Cancer Institute. 92 (2000) 205-216) is categorized in dependency of the volume progression or regression of the tumors (e.g. measured via CT) into four levels: complete response (CR) or partial response (PR), stable disease (SD) and progressive disease (PD) (see Table 1). Furthermore the European Organization for Research and Treatment of Cancer (EORTC) proposed a categorization into four levels in dependency of the metabolism of the tumors measured via 2-[¹⁸F]- Fluoro-2-deoxyglucose positron emission tomography (FDG-PET) (Young H., et al., Eur J Canc 35 (1999) 1773-1782 and Kellof, G. J. , et al, Clin Canc Res 11 (2005) 2785-2808): complete metabolic response (CMR) or partial metabolic response (PMR), stable metabolic disease (SMD) and progressive metabolic disease (PMD) (see Table 2).

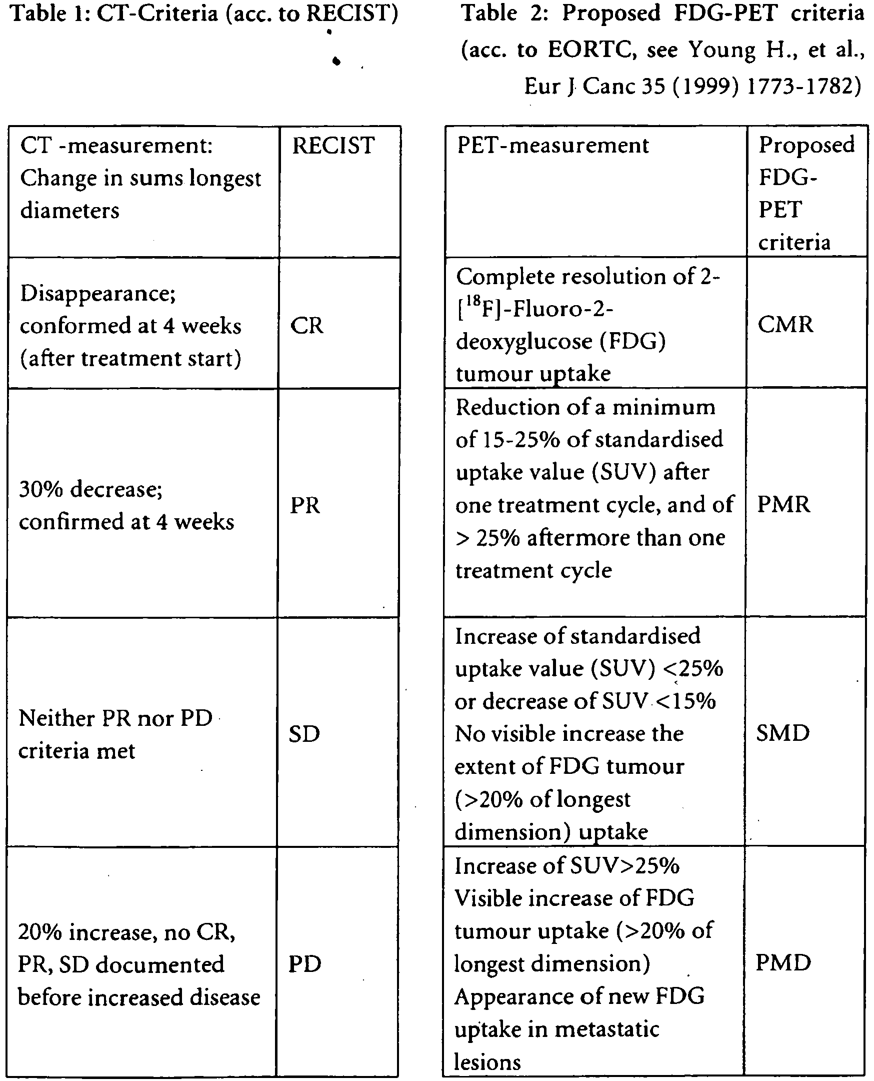

Thus "Response (RE)" and "Non-Response (NR)" according to this invention are most preferably established based on data acquired by the combination of computer tomography (CT) and 2-[18F]-Fluoro-2-deoxyglucose positron emission tomography (FDG-PET) (Kellof, G. J.., et al, Clin Canc Res 11 (2005) 2785- 2808 and Young H., et al., Eur J Canc 35 (1999) 1773-82) using both the RECIST and FDG-PET criteria described above. Accordingly Response (RE) and Non-Response (NR) according to this invention are determined as follows :

**Response (RE)**: CR or PR is established via CT-RECIST criteria (Table 1) and at the same time CMR or PMR is established via FDG-PET (Table 2). Thus Response (RE) means one of the following four cases for combined CT and PET measurement: CR and CMR, PR and PMR, CR and PMR, and PR and CMR.

**Non-Response (NR)**: SD or PD is established via CT-RECIST criteria (Table 1) and at the same time SMD or PMD is established via FDG-PET (Table 2). Thus the following four cases for combined CT and PET measurement signify Non-Response (NR): SD and SMD, SD and PMD, PD and SMD, and PD and PMD.

Usually the response is determined at around 3 to 8 weeks, preferably at around 6 weeks, after treatment start. This response determination is usually repeated at intervals of 4 to 8 weeks, preferably of 6 to 8 weeks. When at the first determination a significant response (RE) was identified, then a relapse (that means a Non-Response (NR) after the first determination) can be determined at earliest at the second response determination. The treatment with the anti-VEGF antibody is started at earliest after the determination of a relapse of the HER2 positive breast cancer. Preferably the treatment with the anti-VEGF antibody of a patient suffering from relapsed HER2 positive breast cancer is started at earliest after 12 weeks, more preferably after 15 weeks, and still more preferably after 18 weeks, from the point of time when the treatment with said anti-HER2 antibody was started.

The term "patient, suffering from relapsed HER2 positive cancer" refers to a patient, in whom Response (RE) is established after the first response determination, and whom in the second or a subsequent response determination Non-Response (NR) is established.

As used herein, the term "patient" preferably refers to a human in need of treatment to treat cancer, or a precancerous condition or lesion. However, the term "patient" can also refer to non-human animals, preferably mammals such as dogs, cats, horses, cows, pigs, sheep and non-human primates, among others, that are in need of treatment.

The term "group" refers to a group of patients as well as a sub-group of patients.

The invention comprises the use of an anti-VEGF antibody for the manufacture of a medicament for preventing or reducing metastasis in a patient, suffering from relapsed HER2 positive cancer, during or after treatment with an anti-HER2 antibody.

A further aspect is an anti-VEGF antibody for preventing or reducing metastasis in a patient, suffering from relapsed HER2 positive breast cancer, during or after treatment with an anti-HER2 antibody. The anti-VEGF antibody is preferably bevacizumab which is preferably administered after a first-line monotherapy with trastuzumab.

In a preferred embodiment, the invention comprises the use of an anti-VEGF antibody for the manufacture of a medicament for preventing or reducing metastasis in a patient, suffering from relapsed HER2 positive cancer, during treatment with an anti-HER2 antibody.

This refers to the the use of an anti-VEGF antibody for the manufacture of a medicament for preventing or reducing metastasis in a patient suffering from relapsed HER2 positive breast cancer comprising co-administering said anti-VEGF antibody and said anti-HER2 antibody to the patient after the relapse of the HER2 positive cancer.

Thus, preferably said medicament is administered during treatment with said anti-HER2 antibody, and preferably following a first-line monotherapy with said anti-HER2 antibody.

In a preferred embodiment, the invention comprises the use of an anti-VEGF antibody for the manufacture of a medicament for preventing or reducing metastasis in a patient, suffering from relapsed HER2 positive breast cancer, after treatment with an anti-HER2 antibody.

This refers to the the use of an anti-VEGF antibody for the manufacture of a medicament for preventing or reducing metastasis in a patient suffering from relapsed HER2 positive breast cancer comprising administering said anti-VEGF antibody alone to the patient after the relapse of the HER2 positive cancer.

Thus, preferably said medicament is administered after treatment with said anti-HER2 antibody.

In a preferred embodiment, the invention comprises the use of an anti-VEGF antibody for the manufacture of a medicament for preventing or reducing metastasis in a patient, suffering from relapsed HER2 positive breast cancer, after a first-line monotherapy with an anti-HER2 antibody.

Thus, preferably said medicament is administered after a first-line monotherapy with said anti-HER2 antibody

Preferably said anti-VEGF antibody binds to the same epitope as bevacizumab.

Preferably said anti-VEGF antibody is bevacizumab.

Preferably said anti-HER2 antibody is trastuzumab.

The term "first-line therapy" as used herein refers to the first type of drug therapy given for the treatment of cancer or metastasis. This can be an adjuvant or neoadjuvant chemotherapy or immunotherapy offered initially following diagnosis and/or surgery. The term "adjuvant chemotherapy or immunotherapy" as used herein refers a treatment after surgery with the intention of prevent cancer from coming back, the term "neoadjuvant chemotherapy or immunotherapy" as used herein refers to a treatment given prior to surgery with the idea of decreasing the tumor size. The term "chemotherapy" as used herein refers to cancer chemotherapy which is the use of chemical or biochemical substances, like cytotoxic drugs such 5-fluoruracil, or targeted therapies with monoclonal antibodies such as trastuzumab, or with kinase inhibitors such as erlotinib, to treat cancer.

The term "first-line monotherapy" as used herein refers to the first-line therapy as defined above with a single chemical or biochemical substance (in contrast to the term "first-line combination therapy" which refers to a first-line therapy with two or more chemical or biochemical substances).

In a preferred embodiment, the invention comprises co-administering said anti-VEGF antibody and said anti-HER2 antibody to the patient.

The term "method for manufacturing a medicament" relates to the manufacturing of a medicament for use in the indication as specified herein and in particular for use in the treatment of tumors, tumor metastases, or cancer in general.

The term "treating" as used herein, unless otherwise indicated, means reversing, alleviating, inhibiting the progress of, or preventing, either partially or completely, the growth of tumors, tumor metastases, or other cancer-causing or neoplastic cells in a patient. The term "treatment" as used herein, unless otherwise indicated, refers to the act of treating.

The phrase "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed an overall beneficial course of action.

It is self-evident that the antibodies are administered to the patient in therapeutically effective amount which is the amount of the subject compound or combination that will elicit the biological or medical response of a tissue, system, animal or human that is being sought by the researcher, veterinarian, medical doctor or other clinician.

The amount of anti-VEGF antibody administration or of anti-VEGF and anti-HER2 antibody co-administration and the timing of administration will depend on the type (species, gender, age, weight, etc.) and condition of the patient being treated and the severity of the disease or condition being treated. Usually typical dosages of anti-VEGF and anti-HER2 antibody like bevacizumab and trastuzumab are used. For example, the dosages for administration of the antibodies according to the invention can be about 1 µg /kg to 50 mg/kg (e.g. 0.1-20 mg/kg) of antibody by one or more separate administrations, or by continuous infusion. A typical daily dosage might range from about 1 µg/kg to about 100 mg/kg. In a preferred aspect, the antibodies are administered every two to three weeks, at a dose ranged from about 1 mg/kg to about 15 mg/kg. A preferred dose for trastuzumab is a loading dose of 4 mg/kg administered as continuous infusion and subsequent 3-weekly infusions of 2 mg/kg to 6 mg/kg, preferably 2 mg/kg, administered as continuous infusion until disease progression is detected. A preferred dose for bevacizumab is 5 mg/kg to 15mg/kg, preferably 5 mg/kg to 10 mg/kg, and more preferred 5 mg/kg , once every 14 days as an IV infusion

The term "during treatment with an anti-HER2 antibody" refers to the "co-administration" or "co-administering" of the anti-VEGF antibody which is administered additionally to the anti-HER2 antibody. The "co-administration" means that the anti-VEGF antibody is administered additionally to the anti-HER2 antibody either simultaneously or sequentially. The coadministration can be simultanous or sequential in either order, wherein preferably there is a time period while both (or all) active agents simultaneously exert their biological activities. When both antibodies are administered simultaneously the dose is administered on the same day in one administration, e.g. during one continuous infusion. When both antibodies are administered sequentially the dose is administered either on the same day in two separate administrations, e.g. two separate continuous infusions, or one of the antibodies is administered on day 1 and the second antibody is administered on day 2 to day 7, preferably on day 2 to 4. The terms "co-administration" or "co-administering" with respect to the maintenance doses of the anti-VEGF antibody and the anti-HER2 antibody mean that the maintenance doses can be either administered simultaneously, e.g. during one continuous infusion, if the treatment cycle is appropriate for both antibodies. Or the maintenance doses are administered sequentially, either within one or several days, e.g. the maintenance dose of the anti HER2-antibody is administered every 3 weeks, and the maintenance dose of the anti-VEGF antibody is administered every 2 weeks. Also other treatment cycles /usually from 1 to 4 weeks, preferably from 2 to 3 weeks, may be used for both antibodies.

The term "after treatment with an anti-HER2 antibody" refers to the administration of the anti-VEGF antibody which is administered after discontinuing the treatment with the anti-HER2 antibody.

The medicament may be useful for reducing metastasis in such a patient , increasing the duration of survival of such a patient, increasing the progression free survival of such a patient, increasing the duration of response, resulting in a statistically significant and clinically meaningful improvement of the treated patient as measured by the duration of survival, progression free survival, response rate or duration of response. The medicament may be useful for increasing the response rate in a group of patients.

In a preferred embodiment, the medicament is useful for reducing metastasis in a patient suffering from relapsed HER2 positive breast cancer during treatment with an anti-HER2 antibody by co-administering said anti-VEGF antibody, preferably bevacizumab, and said anti-HER2 antibody, preferably trastuzumab, to the patient.

The term "metastasis" according to the invention refers to the transmission of cancerous cells from the primary tumor to one or more sites elsewhere in a patient. Means to determine if a cancer has metastasized are known in the art and include bone scan, chest X-ray, CAT scan, MRI scan, and tumor marker tests.

The terms "medicament for preventing metastasis" or "medicament for reducing metastasis" as used herein refer to use of the medicament as a prophylactic agent against metastasis in patient suffering from relapsed HER2 positive cancer in this way inhibiting or reducing a further transmission of cancerous cells from the primary tumor to one or more sites elsewhere in a patient. This means that the metastasis of the primary, metastatic tumor or cancer is prevented, delayed, or inhibited. Preferably the metastasis of the liver is prevented or reduced, which means that metastatic transmission of cancerous cells from the primary tumor to the liver is prevented or reduced.

In the context of this invention, additional other cytotoxic, chemotherapeutic or anti-cancer agents, or compounds that enhance the effects of such agents may be used in the anti-VEGF antibody plus anti-HER2 antibody combination treatment or in the anti-VEGF antibody treatment after failure of a prior therapy, preferably a prior first-line monotherapy, with an anti-HER2 antibody (i.e. in a patient suffering from relapsed HER2 positive cancer after treatment with first-line trastuzumab monotherapy). Preferably the anti-VEGF antibody plus anti-HER2 antibody combination treatment or the anti-VEGF antibody treatment is used without such additional cytotoxic, chemotherapeutic or anti-cancer agents, or compounds that enhance the effects of such agents.

Such agents include, for example: alkylating agents or agents with an alkylating action, such as cyclophosphamide (CTX; e.g. cytoxan^{®}), chlorambucil (CHL; e.g. leukeran^{®}), cisplatin (CisP; e.g. platinol^{®}) busulfan (e.g. myleran^{®}), melphalan, carmustine (BCNU), streptozotocin, triethylenemelamine (TEM), mitomycin C, and the like; anti-metabolites, such as methotrexate (MTX), etoposide (VP16; e.g. vepesid^{®}), 6-mercaptopurine (6MP), 6-thiocguanine (6TG), cytarabine (Ara-C), 5-fluorouracil (5-FU), capecitabine (e.g. Xeloda^{®}), dacarbazine (DTIC), and the like; antibiotics, such as actinomycin D, doxorubicin (DXR; e.g. adriamycin^{®}), daunorubicin (daunomycin), bleomycin, mithramycin and the like; alkaloids, such as vinca alkaloids such as vincristine (VCR), vinblastine, and the like; and other antitumor agents, such as paclitaxel (e.g. taxol^{®}) and paclitaxel derivatives, the cytostatic agents, glucocorticoids such as dexamethasone (DEX; e.g. decadron^{®}) and corticosteroids such as prednisone, nucleoside enzyme inhibitors such as hydroxyurea, amino acid depleting enzymes such as asparaginase, leucovorin and other folic acid derivatives, and similar, diverse antitumor agents. The following agents may also be used as additional agents: arnifostine (e.g. ethyol^{®}), dactinomycin, mechlorethamine (nitrogen mustard), streptozocin, cyclophosphamide, lomustine (CCNU), doxorubicin lipo (e.g. doxil^{®}), gemcitabine (e.g. gemzar^{®}), daunorubicin lipo (e.g. daunoxome^{®}), procarbazine, mitomycin, docetaxel (e.g. taxotere^{®}), aldesleukin, carboplatin, oxaliplatin, cladribine, camptothecin, CPT 11 (irinotecan), 10-hydroxy 7-ethyl-camptothecin (SN38), floxuridine, fludarabine, ifosfamide, idarubicin, mesna, interferon beta, interferon alpha, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, chlorambucil. Preferably the anti-VEGF antibody plus anti-HER2 antibody combination treatment or the anti-VEGF antibody treatment is used without such additional agents.

In the context of this invention, an anti-hormonal agent may be used in the anti-anti-VEGF antibody plus HER2 antibody combination treatment or in the anti-VEGF antibody treatment after failure of a prior therapy, preferably a prior first-line monotherapy, with an anti-HER2 antibody (i.e. in a patient suffering from relapsed HER2 positive cancer). As used herein, the term "anti-hormonal agent" includes natural or synthetic organic or peptidic compounds that act to regulate or inhibit hormone action on tumors. Antihormonal agents include, for example: steroid receptor antagonists, anti-estrogens such as tamoxifen, raloxifene, aromatase inhibiting 4(5)-imidazoles, other aromatase inhibitors, 42-hydroxytamoxifen, trioxifene, keoxifene, LY 117018, onapristone, and toremifene (e.g. Fareston^{®}); anti-androgens such as flutamide, nilutamide, bicalutamide, leuprolide, and goserelin; and pharmaceutically acceptable salts, acids or derivatives of any of the above; agonists and/or antagonists of glycoprotein hormones such as follicle stimulating hormone (FSH), thyroid stimulating hormone (TSH), and luteinizing hormone (LH) and LHRH (leuteinizing hormone-releasing hormone); the LHRH agonist goserelin acetate, commercially available as Zoladex^{®} (AstraZeneca); the LHRH antagonist D-alaninamide N-acetyl-3-(2-naphthalenyl)-D-alanyl-4-chloro-D-phenylalanyl-3-(3-pyridinyl)-D-alanyl-L-seryl-N6-(3-pyridinylcarbonyl)-L-lysyl-N6-(3-pyridinyl-carbonyl)-D-lysyl-L-leucyl-N6-(1-methylethyl)-L-lysyl-L-proline (e.g Antide^{®}, Ares-Serono); the LHRH antagonist ganirelix acetate; the steroidal anti-androgens cyproterone acetate (CPA) and megestrol acetate, commercially available as Megace^{®} (Bristol-Myers Oncology); the nonsteroidal anti-androgen flutamide (2-methyl-N-[4,20-nitro-3-(trifluoromethyl) phenylpropanamide), commercially available as Eulexin^{®} (Schering Corp.); the non-steroidal anti-androgen nilutamide, (5,5-dimethyl-3-[4-nitro-3-(trifluoromethyl-4'-nitrophenyl)-4,4-dimethyl-imidazolidine-dione); and antagonists for other non-permissive receptors, such as antagonists for RAR (retinoic acid receptor), RXR (retinoid X receptor), TR (thyroid receptor), VDR (vitamin-D receptor), and the like. Preferably the anti-VEGF antibody plus anti-HER2 antibody combination treatment or the anti-VEGF antibody treatment is used without such additional anti-hormonal agent.

The use of the cytotoxic and other anticancer agents described above in chemotherapeutic regimens is generally well characterized in the cancer therapy arts, and their use herein falls under the same considerations for monitoring tolerance and effectiveness and for controlling administration routes and dosages, with some adjustments. For example, the actual dosages of the cytotoxic agents may vary depending upon the patient's cultured cell response determined by using histoculture methods. Generally, the dosage will be reduced compared to the amount used in the absence of additional other agents.

Typical dosages of an effective cytotoxic agent can be in the ranges recommended by the manufacturer, and where indicated by in vitro responses or responses in animal models, can be reduced by up to about one order of magnitude concentration or amount. Thus, the actual dosage will depend upon the judgment of the physician, the condition of the patient, and the effectiveness of the therapeutic method based on the in vitro responsiveness of the primary cultured malignant cells or histocultured tissue sample, or the responses observed in the appropriate animal models.

In the context of this invention, additional antiproliferative agents may be used in the anti-VEGF antibody plus anti-HER2 antibody combination treatment or in the anti-VEGF antibody treatment after failure of a prior therapy, preferably a prior first-line monotherapy, with an anti-HER2 antibody, including, for example: Inhibitors of the enzyme farnesyl protein transferase and inhibitors of the receptor tyrosine kinase PDGFR, including the compounds disclosed and claimed in U.S. patent Nos. 6,080,769, 6,194,438, 6,258,824, 6,586,447, 6,071,935, 6,495,564, 6,150,377, 6,596,735 and 6,479,513, and International Patent Publication WO 01/40217. Preferably the anti-VEGF antibody plus anti-HER2 antibody combination treatment or the anti-VEGF antibody treatment is used without such additional antiproliferative agents.

In the context of this invention, an effective amount of ionizing radiation may be carried out and/or a radiopharmaceutical may be used in addition to the anti-VEGF antibody plus anti-HER2 antibody combination treatment or in the anti-VEGF antibody treatment after failure of a prior therapy, preferably a prior first-line monotherapy, with an anti-HER2 antibody (i.e. in a patient suffering from relapsed HER2 positive cancer). The source of radiation can be either external or internal to the patient being treated. When the source is external to the patient, the therapy is known as external beam radiation therapy (EBRT). When the source of radiation is internal to the patient, the treatment is called brachytherapy (BT). Radioactive atoms for use in the context of this invention can be selected from the group including, but not limited to, radium, cesium-137, iridium-192, americium-241, gold-198, cobalt-57, copper-67, technetium-99, iodine-123, iodine-131, and indium-111. Where the EGFR kinase inhibitor according to this invention is an antibody, it is also possible to label the antibody with such radioactive isotopes. Preferably the anti-VEGF antibody plus anti-HER2 antibody combination treatment or the anti-VEGF antibody treatment is used without such ionizing radiation.

Radiation therapy is a standard treatment for controlling unresectable or inoperable tumors and/or tumor metastases. Improved results have been seen when radiation therapy has been combined with chemotherapy. Radiation therapy is based on the principle that high-dose radiation delivered to a target area will result in the death of reproductive cells in both tumor and normal tissues. The radiation dosage regimen is generally defined in terms of radiation absorbed dose (Gy), time and fractionation, and must be carefully defined by the oncologist. The amount of radiation a patient receives will depend on various considerations, but the two most important are the location of the tumor in relation to other critical structures or organs of the body, and the extent to which the tumor has spread. A typical course of treatment for a patient undergoing radiation therapy will be a treatment schedule over a 1 to 6 week period, with a total dose of between 10 and 80 Gy administered to the patient in a single daily fraction of about 1.8 to 2.0 Gy, 5 days a week. In a preferred embodiment of this invention there is synergy when tumors in human patients are treated with the combination treatment of the invention and radiation. In other words, the inhibition of tumor growth by means of the agents comprising the combination or single therapy of the invention is enhanced when combined with radiation, optionally with additional chemotherapeutic or anticancer agents. Parameters of adjuvant radiation therapies are, for example, contained in International Patent Publication WO 99/60023.

The antibodies are administered to a patient according to known methods, by intravenous administration as a bolus or by continuous infusion over a period of time, by intramuscular, intraperitoneal, intracerobrospinal, subcutaneous, intra-articular, intrasynovial, or intrathecal routes. Intravenous or subcutaneous administration of the antibodies is preferred.

Also disclosed is an article of manufacture comprising a container, a composition within the container comprising an anti-VEGF antibody and a package insert instructing the user of the composition to administer said anti-VEGF antibody to a patient suffering from relapsed HER2 positive cancer during or after treatment with an anti-HER2 antibody

The term "package insert" refers to instructions customarily included in commercial packages of therapeutic products, which may include information about the indications, usage, dosage, administration, contraindications and/or warnings concerning the use of such therapeutic products.

The article of manufacture containers may further include a pharmaceutically acceptable carrier. The article of manufacture may further include a sterile diluent, which is preferably stored in a separate additional container.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention are contemplated. Supplementary active compounds can also be incorporated into the compositions.

The following examples and figures are provided to aid the understanding of the present invention, the true scope of which is set forth in the appended claims.

### Description of the Figures

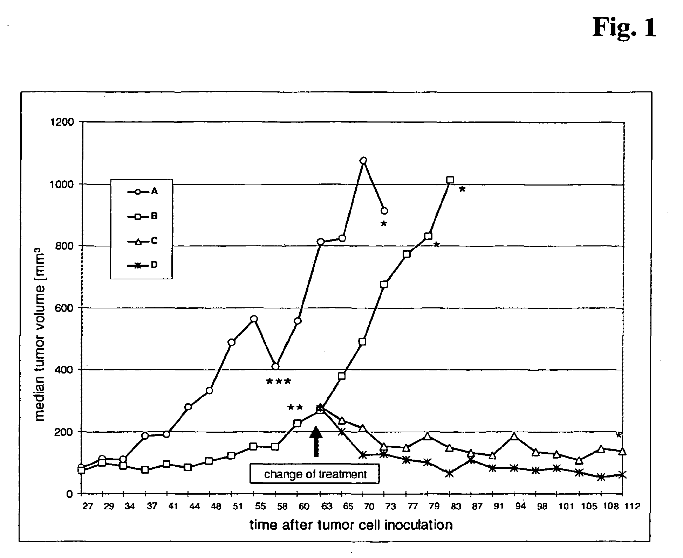

- **Figure 1**: Antitumor activity of a) combined trastuzumab and bevacizumab treatment and b) bevacizumab treatment alone on tumor growth after trastuzumab treatment failure. Mean values of tumor volume (mm³) plotted on the y-axis; number of days after injection of tumor cells plotted on the x-axis. A)Vehicle (*circles*), B) trastuzumab at loading dose of 30mg/kg and maintenance dose of 15mg/kg once weekly (*squares*). At day 60 the animals of Group B were divided into three further Groups B' (*squares after day 60*), C and D. The treatment with trastuzumab alone was maintained only for Group B' with once weekly doses of 15 mg/kg (maintenance dose).C) from day 61 trastuzumab maintenance dose of 15mg/kg once weekly in combination with an additional bevacizumab treatment dose at 5mg/kg twice weekly (*triangles)* and D) from day 61 bevacizumab at 5mg/kg twice weekly when treatment with trastuzumab is discontinued (crosses). Time points when one mouse (*) or more mice (***) were sacrified based on termination criteria are indicated by one or more asterisks (*).

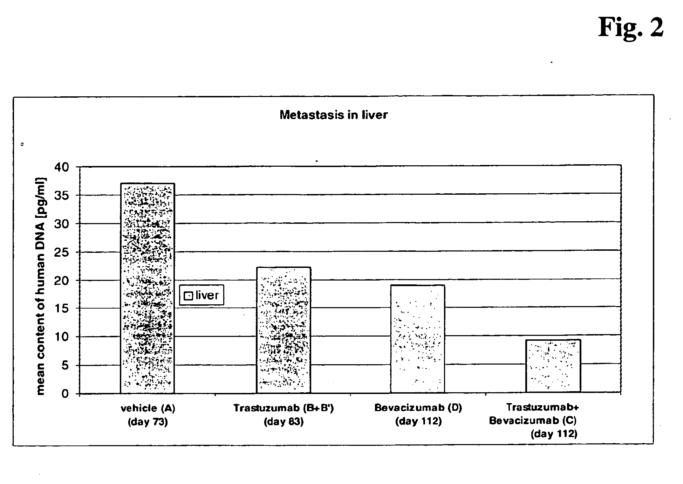

- **Figure 2**: Effect of (A) no treatment-vehicle group, (B) trastuzumab treatment alone (until day 83), (C) (combined trastuzumab and bevacizumab treatment (day 61 - day 112) after trastuzumab treatment alone (day 27 - day 60) and (D) bevacizumab treatment alone (day 61 - day 112) after trastuzumab treatment alone (day 27 - day 60) on liver metastasis. Mean value of human Alu DNA sequence (pg/ml) quantitated from liver tissue using real-time PCR and plotted on the y-axis.

### Experimental Procedures

### Introduction

The current study examined the antitumor activity of a) the combination of trastuzumab and bevacizumab and b) the treatment with bevacizumab alone, after the failure of trastuzumab treatment alone in human breast xenograft model. Further aims of the study were to examine the effects of treatment on metastasis.

### Test agents

Trastuzumab was provided as a 25mg/ml stock solution in Histidine-HCl, alpha-alpha Trehalose (60mM), 0.01% Polysorb, pH 6.0 (Herceptin^{®}). Bevacizumab was provided as a 25mg/ml stock solution in Na-phosphate, alpha-alpha Trehalose (60mM), 0.01% Polysorb, pH 6.0 (Avastin^{®}). Both solutions were diluted appropriately in PBS for injections.

### Cell lines and culture conditions

The human breast cancer cell line KPL-4 has been established from the malignant pleural effusion of a breast cancer patient with an inflammatory skin metastasis and overexpresses ErbB family receptors. (Kurebayashi et al., Br. J. Cancer 79 (1999) 707-17) Tumor cells are routinely cultured in DMEM medium (PAA Laboratories, Austria) supplemented with 10 % fetal bovine serum (PAA) and 2 mM L-glutamine (Gibco) at 37°C in a water-saturated atmosphere at 5 % CO2. Culture passage is performed with trypsin / EDTA 1x (PAA) splitting twice / week. Cell passage P6 was used for in vivo study.

### Animals

SCID beige (C.B.-17) mice; age 10-12 weeks; body weight 18-20 g (Charles River, Sulzfeld, Germany) are maintained under specific-pathogen-free condition with daily cycles of 12 h light /12 h darkness according to international guidelines (GV-Solas; Felasa; TierschG). After arrival, animals are housed in the quarantine part of the animal facility for one week to get accustomed to new environment and for observation. Continuous health monitoring is carried out on regular basis. Diet food (Alltromin) and water (acidified pH 2.5-3) are provided ad libitum.

### Tumor growth inhibition studies in vivo

Tumor cells were harvested (trypsin-EDTA) from culture flasks (Greiner TriFlask) and transferred into 50 ml culture medium, washed once and resuspended in PBS. After an additional washing step with PBS and filtration (cell strainer; Falcon 100µm) the final cell titer was adjusted to 0.75 x 10⁸ / ml. Tumor cell suspension was carefully mixed with transfer pipette to avoid cell aggregation. Anesthesia was performed using a Stephens inhalation unit for small animals with preincubation chamber (plexiglas), individual mouse nose-mask (silicon) and Isoflurane (Pharmacia-Upjohn, Germany) in a closed circulation system. Two days before injection the fur of the animals was shaved. For intra mammary fat pad (i. m.f.p.) injection, cells were injected orthotopically at a volume of 20 µl into the right penultimate inguinal mammary fat pad of each anesthetized mouse. For the orthotopic implantation, the cell suspension was injected through the skin under the nipple. Tumor cell injection corresponds to day 1 of the experiment.

### Monitoring

Animals were controlled daily for detection of clinical symptoms of adverse effects. For monitoring throughout the experiment, the body weight of the animals was documented two times weekly and the tumor volume was measured by caliper twice weekly. Primary tumor volume was calculated according to NCI protocol (TV = 1/2ab2, where a and b are long and short diameters of tumor size in mm, Teicher, B., Anticancer drug development guide, Humana Press, 5, (1997) 92). Calculation values were documented as mean and standard deviation.

### Treatment of animals

Tumor-bearing mice were randomized when the tumor volume was roughly 100 mm³ (n=10 for each group). Each group was closely matched before treatment, which began 27 days after tumor cell injection. Group A: Vehicle group - received 10 ml/kg PBS buffer intraperitoneally (i.p.) once weekly. Group B: Trastuzumab was administered i.p. at a loading dose of 30 mg/kg, followed by once weekly doses of 15 mg/kg (maintenance dose). At day 60 the animals of Group B were divided into three further Groups B', C and D. The treatment with trastuzumab alone was maintained only for Group B' with once weekly doses of 15 mg/kg (maintenance dose). Group C: At day 61, treatment for Group C was switched to a combination treatment of trastuzumab (15 mg/kg once weekly i.p.) and bevacizumab (5 mg/kg twice weekly i.p.). Group D: At day 61, treatment for Group D was switched to a monotherapy with bevacizumab (5 mg/kg twice weekly i.p.) while treatment with trastuzumab was discontinued.

### Evaluation of metastasis

Spread of tumor cells into the lung was determined in sacrificed animals. Metastasis was measured according to Schneider, T., et al., Clin. Exp. Metas. 19 (2002) 571-582. Briefly, lung tissue was harvested and human Alu sequences were quantified by real-time PCR. Higher human DNA levels, quantified by real-time PCR, correspond to higher levels of metastasis.

### Results

The effect of treatment on primary tumor growth is shown in Figure 1 and table 1. Tumors in the vehicle group (Group A) grew rapidly and mice were sacrificed 73 days after injection of tumor cells because of ulceration of tumors and the development of clinical symptoms. Treatment with trastuzumab (Group B) suppressed tumor growth significantly; however, tumors started to regrow around day 50. The change to combination treatment with trastuzumab and bevacizumab (Group C) as well as the switch to bevacizumab monotherapy (Group D) beginning at day 61, both resulted in complete inhibition of tumor growth during the duration of the experiment (day 112) and treatment was well tolerated.

**Table 1:**

| | | | | | | | | |
|---|---|---|---|---|---|---|---|---|
| **Antitumor activity of a) combined trastuzumab and bevacizumab and b) bevacizumab treatment alone on tumor growth after trastuzumab treatment failure (data for** **figure 1****). Mean tumor volume in mm³ is reported and the standard deviation (SD).** | | | | | | | | |

| Day | Vehicle (A) | SD | Trastuzumab (B+B') | SD | Change from Trastuzumab to Trastuzumab + Bevacizumab (C) | SD | Change from Trastuzumab to Bevacizumab monotherapy (D) | SD |
|---|---|---|---|---|---|---|---|---|
| 27 | 85 | 27 | 81 | 29 | | | | |
| 29 | 115 | 42 | 106 | 36 | | | | |
| 34 | 136 | 66 | 100 | 49 | | | | |
| 37 | 193 | 108 | 97 | 70 | | | | |
| 41 | 235 | 163 | 133 | 100 | | | | |
| 44 | 335 | 220 | 139 | 128 | | | | |
| 48 | 406 | 309 | 172 | 181 | | | | |
| 51 | 591 | 463 | 201 | 203 | | | | |
| 55 | 690 | 479 | 263 | 286 | | | | |
| 58 | 565 | 333 | 315 | 383 | | | | |
| 60 | 729 | 402 | 393 | 426 | | | | |
| 63 | 911 | 391 | 493 | 531 | 407 | 263 | 427 | 365 |
| 65 | 898 | 313 | 585 | 582 | 350 | 210 | 306 | 220 |
| 70 | 1213 | 440 | 798 | 776 | 190 | 45 | 180 | 142 |
| 73 | 1015 | 330 | 961 | 841 | 149 | 44 | 154 | 112 |
| 77 | | | 861 | 418 | 146 | 45 | 129 | 78 |
| 79 | | | 896 | 434 | 159 | 92 | 127 | 84 |
| 83 | | | 1034 | 485 | 158 | 148 | 97 | 80 |
| 87 | | | | | 193 | 228 | 95 | 57 |
| 91 | | | | | 159 | 166 | 100 | 82 |
| 94 | | | | | 225 | 292 | 120 | 106 |
| 98 | | | | | 242 | 340 | 112 | 95 |
| 101 | | | | | 154 | 160 | 120 | 108 |
| 105 | | | | | 119 | 109 | 92 | 85 |
| 108 | | | | | 175 | 157 | 104 | 105 |
| 112 | | | | | 122 | 68 | 110 | 103 |

The effect of treatment on liver metastasis is shown in figure 2 and table 2. The combination of trastuzumab and bevacizumab after trastuzumab treatment failure resulted in a sharp decrease of metastasis. Levels of human Alu sequences (correlating to invasion of tumor cells into secondary tissue) are significantly lower in animals treated with a combination therapy for 31 days starting at day 61 compared to vehicle treated animals that were sacrificed at day 73. Also metastasis was suppressed in trastuzumab or bevacizumab monotherapy treated animals sacrificed on day 83 or 112 respectively This surprising effect on metastasis is in contrast with the effect seen with cytotoxic drugs (Geldof et al., Anticancer Res. 8 (1988) 1335-40, Murphy, J., Clin. Oncol. 11 (1993) 199-201, and De Larco et al., Cancer Res. 61 (2001) 2857-61).

**Table 2:**

| | | | | |
|---|---|---|---|---|
| **Effect of treatment on liver metastasis. Alu DNA was quantified by real-time PCR and is reported for each animal.** | | | | |

| | Vehicle (A) (day 73) | Trastuzumab (B+B') (day 83) | Bevacizumab (D) (day 112) | Trastuzumab + Bevacizumab (C) (day 112) |
|---|---|---|---|---|
| human DNA [pg/ml] | 41.750 | 21.000 | 12.250 | 7.155 |
| | 51.400 | 10.550 | 7.405 | 6.785 |
| | 54.500 | 26.600 | 45.600 | 15.500 |
| | 19.300 | 12.250 | 29.200 | 8.040 |
| | 6.545 | 37.900 | 7.640 | 8.305 |
| | 48.550 | 25.050 | 22.900 | |
| | | | 7.740 | |
| Mean | 37.008 | 22.225 | 18.962 | 9.157 |

## Claims

1. Use of an anti-VEGF antibody for the manufacture of a medicament for preventing or reducing metastasis in a patient, suffering from relapsed HER2 positive breast cancer, during or after treatment with an anti-HER2 antibody, wherein said relapsed HER2 positive breast cancer refers to the uncontrolled growth of abnormal cells **characterized by** HER2 protein overexpression in tumor patients who initially responded to previous therapy with an anti-HER2 antibody, but in whom the therapeutic response was not maintained during treatment with said anti-HER2 antibody.

2. Use according to claim 1 **characterized in that** said medicament is for administration to the patient during treatment with an anti-HER2 antibody.

3. Use according to claim 2 **characterized in that** said medicament is for administration to the patient following a first-line monotherapy with an anti-HER2 antibody.

4. Use according to claim 1 **characterized in that** said medicament is for administration to the patient after treatment with an anti-HER2 antibody.

5. Use according to claim 4 **characterized in that** said medicament is for administration to the patient after a first-line monotherapy with an anti-HER2 antibody.

6. Use according to any one of claims 1 to 5, **characterized in that** said anti-VEGF antibody is bevacizumab.

7. The use of any one of claims 1 to 6, **characterized in that** said anti-HER2 antibody is trastuzumab.

8. An anti-VEGF antibody for use in a method of preventing or reducing metastasis in a patient, suffering from relapsed HER2 positive breast cancer, during or after treatment with an anti-HER2 antibody, wherein said relapsed HER2 positive breast cancer refers to the uncontrolled growth of abnormal cells **characterized by** HER2 protein overexpression in tumor patients who initially responded to previous therapy with an anti-HER2 antibody, but in whom the therapeutic response was not maintained during treatment with said anti-HER2 antibody.

9. The anti-VEGF antibody for use according to claim 8, which is for administration to the patient during treatment with an anti-HER2 antibody.

10. The anti-VEGF antibody for use according to claim 9, which is for administration to the patient following a first-line monotherapy with an anti-HER2 antibody.

11. The anti-VEGF antibody for use according to claim 8, which is for administration to the patient after treatment with an anti-HER2 antibody.

12. The anti-VEGF antibody for use according to claim 11, which is for administration to the patient after a first-line monotherapy with an anti-HER2 antibody.

13. The anti-VEGF antibody for use according to any one of claims 8-12, which is bevacizumab.

14. The anti-VEGF antibody for use according to any one of claims 8-13, **characterized in that** said anti-HER2 antibody is trastuzumab.

15. The anti-VEGF antibody according to claim 8 **characterized in that** said anti-VEGF is bevacizumab which is for administration after a first-line monotherapy with trastuzumab.

16. Use according to claim 1 **characterized in that** said anti-VEGF is bevacizumab and **in that** said medicament is for administration after a first-line monotherapy with trastuzumab.

## Patentansprüche

1. Verwendung eines Anti-VEGF-Antikörpers für die Herstellung eines Medikaments zur Vorbeugung oder Reduktion von Metastasen in einem Patienten, der an einem Rückfall von HER2-positivem Brustkrebs leidet, während oder nach der Behandlung mit einem Anti-HER2-Antikörper, wobei der Rückfall von HER2-positivem Brustkrebs sich auf das unkontrollierte Wachstum von anormalen Zellen bezieht, **gekennzeichnet durch** HER2-Protein-Überexpression in Tumorpatienten, die anfänglich auf eine frühere Therapie mit einem Anti-HER2-Antikörper reagiert haben, aber bei denen die Therapieantwort während der Behandlung mit dem Anti-HER2-Antikörper nicht erhalten blieb.

2. Verwendung gemäß Anspruch 1, **dadurch gekennzeichnet, dass** das Medikament für die Verabreichung an den Patienten während der Behandlung mit einem Anti-HER2-Antikörper ist.

3. Verwendung gemäß Anspruch 2, **dadurch gekennzeichnet, dass** das Medikament für die Verabreichung an den Patienten im Anschluss an eine First-Line-Monotherapie mit einem Anti-HER2-Antikörper ist.

4. Verwendung gemäß Anspruch 1, **dadurch gekennzeichnet, dass** das Medikament für die Verabreichung an den Patienten nach der Behandlung mit einem Anti-HER2-Antikörper ist.

5. Verwendung gemäß Anspruch 4, **dadurch gekennzeichnet, dass** das Medikament für die Verabreichung an den Patienten nach einer First-Line-Monotherapie mit einem Anti-HER2-Antikörper ist.

6. Verwendung gemäß einem der Ansprüche 1 bis 5, **dadurch gekennzeichnet, dass** der Anti-VEGF-Antikörper Bevacizumab ist.

7. Verwendung gemäß einem der Ansprüche 1 bis 6, **dadurch gekennzeichnet, dass** der Anti-HER2-Antikörper Trastuzumab ist.

8. Anti-VEGF-Antikörper zur Verwendung in einem Verfahren zur Vorbeugung oder Reduktion von Metastasen in einem Patienten, der an einem Rückfall von HER2-positivem Brustkrebs leidet, während oder nach der Behandlung mit einem Anti-HER2-Antikörper, wobei der Rückfall von HER2-positivem Brustkrebs sich auf das unkontrollierte Wachstum von anormalen Zellen bezieht, **gekennzeichnet durch** HER2-Protein-Überexpression in Tumorpatienten, die anfänglich auf eine frühere Therapie mit einem Anti-HER2-Antikörper reagiert haben, aber bei denen die Therapieantwort während der Behandlung mit dem Anti-HER2-Antikörper nicht erhalten blieb.

9. Anti-VEGF-Antikörper zur Verwendung gemäß Anspruch 8, welcher für die Verabreichung an den Patienten während der Behandlung mit einem Anti-HER2-Antikörper ist.

10. Anti-VEGF-Antikörper zur Verwendung gemäß Anspruch 9, welcher für die Verabreichung an den Patienten im Anschluss an eine First-Line-Monotherapie mit einem Anti-HER2-Antikörper ist.

11. Anti-VEGF-Antikörper zur Verwendung gemäß Anspruch 8, welcher für die Verabreichung an den Patienten nach der Behandlung mit einem Anti-HER2-Antikörper ist.

12. Anti-VEGF-Antikörper zur Verwendung gemäß Anspruch 11, welcher für die Verabreichung an den Patienten nach einer First-Line-Monotherapie mit einem Anti-HER2-Antikörper ist.

13. Anti-VEGF-Antikörper zur Verwendung gemäß einem der Ansprüche 8-12, welcher Bevacizumab ist.

14. Anti-VEGF-Antikörper zur Verwendung gemäß einem der Ansprüche 8-13, **dadurch gekennzeichnet, dass** der Anti-HER2-Antikörper Trastuzumab ist.

15. Anti-VEGF-Antikörper zur Verwendung gemäß Anspruch 8, **dadurch gekennzeichnet, dass** der Anti-VEGF-Antikörper Bevacizumab ist, das für die Verabreichung nach einer First-Line-Monotherapie mit Trastuzumab ist.

16. Verwendung gemäß Anspruch 1, **dadurch gekennzeichnet, dass** der Anti-VEGF-Antikörper Bevacizumab ist und dass das Medikament für die Verabreichung nach einer First-Line-Monotherapie mit Trastuzumab ist.

## Revendications

1. Utilisation d'un anticorps anti-VEGF pour la fabrication d'un médicament destiné à prévenir ou à réduire les métastases chez un patient souffrant d'un cancer du sein HER2-positif ayant récidivé, pendant ou après un traitement avec un anticorps anti-HER2, où ledit cancer du sein HER2-positif ayant récidivé désigne la croissance incontrôlée de cellules anormales **caractérisées par** une surexpression de la protéine HER2 chez des patients souffrant d'une tumeur, qui ont répondu initialement à une thérapie antérieure avec un anticorps anti-HER2, mais chez qui la réponse thérapeutique n'était pas maintenue pendant le traitement avec ledit anticorps anti-HER2.

2. Utilisation selon la revendication 1, **caractérisée en ce que** ledit médicament est destiné à une administration au patient pendant le traitement avec un anticorps anti-HER2.

3. Utilisation selon la revendication 1, **caractérisée en ce que** ledit médicament est destiné à une administration au patient suivant une monothérapie de première intention avec un anticorps anti-HER2.

4. Utilisation selon la revendication 1, **caractérisée en ce que** ledit médicament est destiné à une administration au patient après le traitement avec un anticorps anti-HER2.

5. Utilisation selon la revendication 4, **caractérisée en ce que** ledit médicament est destiné à une administration au patient après une monothérapie de première intention avec un anticorps anti-HER2.

6. Utilisation selon l'une quelconque des revendications 1 à 5, **caractérisée en ce que** ledit anticorps anti-VEGF est le bévacizumab.

7. Utilisation selon l'une quelconque des revendications 1 à 6, **caractérisée en ce que** ledit anticorps anti-HER2 est le trastuzumab.

8. Anticorps anti-VEGF à utiliser dans un procédé de prévention ou de réduction des métastases chez un patient souffrant d'un cancer du sein HER2-positif ayant récidivé, pendant ou après un traitement avec un anticorps anti-HER2, où ledit cancer du sein HER2-positif ayant récidivé désigne la croissance incontrôlée de cellules anormales **caractérisées par** une surexpression de la protéine HER2 chez des patients souffrant d'une tumeur, qui ont répondu initialement à une thérapie antérieure avec un anticorps anti-HER2, mais chez qui la réponse thérapeutique n'était pas maintenue pendant le traitement avec ledit anticorps anti-HER2.

9. Anticorps anti-VEGF à utiliser selon la revendication 8, qui est destiné à une administration au patient pendant le traitement avec un anticorps anti-HER2.

10. Anticorps anti-VEGF à utiliser selon la revendication 9, qui est destiné à une administration au patient suivant une monothérapie de première intention avec un anticorps anti-HER2.

11. Anticorps anti-VEGF à utiliser selon la revendication 8, qui est destiné à une administration au patient après le traitement avec un anticorps anti-HER2.

12. Anticorps anti-VEGF à utiliser selon la revendication 11, qui est destiné à une administration au patient après une monothérapie de première intention avec un anticorps anti-HER2.

13. Anticorps anti-VEGF à utiliser selon l'une quelconque des revendications 8-12, qui est le bévacizumab.

14. Anticorps anti-VEGF à utiliser selon l'une quelconque des revendications 8-13, **caractérisé en ce que** ledit anticorps anti-HER2 est le trastuzumab.

15. Anticorps anti-VEGF à utiliser selon la revendication 8, **caractérisé en ce que** ledit anti-VEGF est le bévacizumab qui est destiné à une administration après une monothérapie de première intention avec du trastuzumab.

16. Utilisation selon la revendication 1, **caractérisé en ce que** ledit anti-VEGF est le bévacizumab et **en ce que** ledit médicament est destiné à une administration après une monothérapie de première intention avec du trastuzumab.
